# EUROPEAN PATENT SPECIFICATION

(11) **EP 1 177 309 B1**
(45) Date of publication and mention of the grant of the patent: **18.02.2004**
(21) Application number: 99920387.0
(22) Date of filing: 07.05.1999
(51) Int. Cl.: C12P 7/06

(54) **CLOSTRIDIUM STRAINS WHICH PRODUCE ETHANOL FROM SUBSTRATE-CONTAINING GASES**
AUS SUBSTRATHALTIGEN GASGEMISCHEN AETHANOL ERZEUGENDE CLOSTRIDIUM STAMME
SOUCHES DE CLOSTRIDIUM PRODUISANT DE L'ETHANOL A PARTIR DE GAZ DE COMBUSTION

(43) Date of publication of application: 06.02.2002
(73) Proprietor: Emmaus Foundation, Inc., Fayetteville, Arkansas 72701 (US)
(72) Inventor: GADDY, James, L., Fayetteville, AR 72703 (US); Dr. Guangjiong Chen, Ballwin, MO 63017 (US); Youhong Li, . (US)
(74) Representative: Hale, Stephen Geoffrey
(86) International application number: PCT/US1999/010022
(87) International publication number: WO 2000/068407

(56) References cited:
- US-A- 5 173 429
- US-A- 5 593 886
- US-A- 5 807 722

## Description

This invention was supported by the U.S. Department of Energy, Grant Nos. DE-FCO2-90CE40939 and DE-FC04-94AL98770. The U.S. Government has certain rights in this invention.

### Field of the Invention

The present invention is directed to biological microorganisms useful in the anaerobic fermentation of certain gaseous substrates, particularly those from industrial processes, to desirable products.

### Background of the Invention

Various industrial processes generate worldwide massive amounts of atmospheric pollutants and greenhouse gases. Waste carbon monoxide, carbon dioxide and hydrogen gases are generated by petroleum refining. Carbon monoxide and/or hydrogen are also generated by carbon black manufacture and coke production, by the ammonia industry, by steel mills, by the sulfate pulping of wood and by the adipic acid industry. These waste gas streams are generally burned for fuel value or flared. In many cases, these gases are discharged directly to the atmosphere, placing a heavy pollution burden on the environment.

There are a variety of microorganisms that are potentially useful in fermentation processes, including anaerobic bacteria such as Clostridium ljungdahlii strain PETC or ERI2, among others [See e.g., US Patent Nos. 5,173,429; 5,593,886 and 5,821,111; and references cited therein; see also International Patent Application No. PCT/US96/11146, published as WO98/00558 on January 8, 1998]. However, there remains a continuing need in the art for the development and/or isolation of new microorganisms, which are able to utilize the above-described waste gases and produce useful products therefrom.

### Summary of the Invention

In one aspect, the invention provides a biologically pure culture of a novel microorganism, O-52, which under anaerobic conditions, has the ability to produce ethanol upon fermentation in an aqueous nutrient medium.

In another aspect, the invention provides a biologically pure culture of a novel microorganism, C-01, which under anaerobic conditions, has the ability to produce ethanol upon fermentation in an aqueous nutrient medium.

Other aspects and advantages of the present invention will become apparent from the following detailed description.

### Detailed Description of the Invention

The present invention provides two anaerobic strains of *Clostridium ljungdahlii* bacteria, isolated from contaminants with which they are associated in nature, and which are useful in processes for the fermentative conversion of certain gas streams or waste gases to ethanol.

### A. An Exemplary Fermentation Process

The term "waste gas", "synthesis gas" or "substrate-containing gas" as used herein means carbon monoxide and/or carbon dioxide and/or water and/or hydrogen mixed with other elements or compounds, including e.g., nitrogen and methane, in a gaseous state. Such gases or streams are typically released or exhausted to the atmosphere either directly or through combustion. Normally, release takes place under standard smokestack temperatures and pressures. In one embodiment, the substrate-containing gas contains carbon monoxide and water. In another embodiment, the substrate-containing gas contains carbon dioxide and hydrogen. Accordingly, the microorganisms of the present invention are useful in processes for converting these atmospheric pollutants into useful products, primarily ethanol.

An exemplary fermentation conversion process in which the microorganisms of the present invention can be employed is as follows. It is understood by one of skill in the art that these microorganisms may be employed in other processes, and that variables in the processes can be adjusted to provide a preferred result. Such variable factors include nutrient constituents and concentrations, medium, pressure, temperature, gas flow rate, liquid flow rate, reaction pH, agitation rate (if utilizing a Continuously Stirred Tank Reactor), inoculum level, maximum substrate (introduced gas) concentrations to avoid inhibition, and maximum product concentrations to avoid inhibition. In the embodiment of the process described below, it is preferred that the process is conducted at higher than 1 atmosphere. Preferably, it is preferred that it be conducted at pressures up to 320 atmospheres, and more preferably up to 20 atmospheres, and most preferably up to 15 atmospheres.

Generally in a process to produce ethanol from a waste or substrate-containing gas, a first step in the conversion process is the preparation of nutrient media for the anaerobic bacteria. One such desirable media is disclosed specifically in Example 2 below. In a desirable embodiment, basal medium is used without yeast extract and trypticase. However, the contents of the nutrient media can vary as desired by one of skill in the art. The nutrients are constantly fed to a bioreactor or fermenter which can consist of one or more vessels and/or towers of a type which includes the Continuously Stirred Reactor (CSTR), Immobilized Cell Reactor (ICR), Trickle Bed Reactor (TBR), Bubble column, Gas Lift Fermenters, or other suitable fermentation reactor. Within the bioreactor resides the microorganism culture, which can be either single species or a mixture of O-52, C-01 and possibly other known anaerobic bacterial species, capable of producing ethanol in place of acetic acid/acetate. In general, a low fermentation pH of 4.0 - 5.5 is required.

For the CSTRs, TBRs, Bubble Columns and Gas Lift Fermenters, these bacteria live dispersed throughout the liquid phase of the reactor, but for ICRs, the bacteria adhere to an internal packing medium. This packing medium must provide maximal surface area, high mass transfer rate, low pressure drop, even gas and liquid distribution, and must minimize plugging, fouling, nesting and wall channeling. Examples of such medium materials are ceramic Berl saddles, Raschig rings or other high performance packings.

The substrate containing gases are continuously introduced into the bioreactor and retained in the bioreactor for a period of time which maximizes efficiency of the process. Exhaust gases containing inert substances and unreacted substrate gases are then released. The liquid effluent is passed to a centrifuge, hollow fiber membrane, or other filtration device to separate out microorganisms that are entrained. These microorganisms can be returned to the bioreactor to maintain a high cell concentration which yields a faster reaction rate (cell recycle). Cell recycle may be used to enhance the cell concentration in the reactor, but this operation is not required to make the process work. Optionally this latter cell recycle step can be omitted.

A next step in the process is separation of ethanol from the permeate or centrifugate. The permeate from the cell recycle apparatus containing dilute ethanol in medium is passed to an extraction chamber where it is contacted with a solvent. The solvent should have a high distribution coefficient for ethanol, a high recovery factor, low toxicity to humans, low toxicity to the bacteria, immiscibility with water, an appropriately high boiling point, and should form no emulsion with the bioreactor constituents. The distribution of solute between solvent and aqueous phases will determine the thermodynamic feasibility and the amount of solvent required to remove the ethanol. Typical solvents include secondary and tertiary amines in a suitable solvent, tributyl phosphate, ethyl acetate, tri-octyl phosphine oxide and related compounds in a suitable co-solvent, long chain alcohols, hexane, cyclohexane, chloroform, and tetrachloroethylene.

The nutrients and materials in the aqueous phase pass back to the bioreactor and the solvent/ethanol/water solution passes to a distillation column, where they are heated to a sufficient temperature to separate the solvent from the ethanol and water. The solvent passes from the distillation column through a cooling chamber to lower the temperature to the optimum temperature for extraction, then back to the extraction chamber for reuse. The ethanol and water solution passes to a final distillation column where the ethanol is separated from the water and removed. Ninety-five percent ethanol exits the top of the distillation column and water (spent medium) exits the bottom of the column. The spent medium is sent back to the reactor as water recycle. The 95% ethanol is sent to a molecular sieve system to produce anhydrous ethanol. The water is recirculated for nutrient preparation.

### B. The Novel Microorganisms

Both microorganisms of this invention, O-52 and C-01, have been deposited pursuant to the appropriate regulations of the Budapest Treaty on the International Recognition of The Deposit of Microorganisms for the Purposes of Patent Procedure. *C. ljungdahlii*, strain O-52 was deposited with the American Type Culture Collection, 10801 University Boulevard, Manassas, VA 20110-2209, USA (formerly located at 12301 Parklawn Drive, Rockville, MD 20852, USA) on June 27, 1997 under Accession No. 55989. *C. ljungdahlii*, strain C-01 was deposited with the American Type Culture Collection, 10801 University Boulevard, Manassas, VA 20110-2209, USA (formerly located at 12301 Parklawn Drive, Rockville, MD 20852, USA) on June 27, 1997 under Accession No. 55988. Neither strain has been released to the public as of this filing date.

These microorganism are useful in fermentation processes for producing ethanol from waste gases from industrial streams, as well as other synthesis gases. In fact, these new strains of anaerobic bacteria can participate in methods for conversion of the gaseous substrates to ethanol, as described above, with high efficiency.

These anaerobic bacterial strains, O-52 and C-01, behave quite similarly, showing a high tolerance to both CO and ethanol. Both microorganisms use CO, CO₂ and H₂, and produce similar concentrations of ethanol with low by-product acetic acid concentrations.

*C. ljungdahlii* O-52 is capable of producing ethanol instead of acetic acid as a product from a substrate-containing gas conversion. This was accomplished in processes such as those described above, preferably by utilizing basal medium without yeast extract and trypticase. O-52 has been observed to produce ethanol in the CSTR with only small amounts of acetic acid as a by-product. In one embodiment of the process described above employing O-52 as the microorganism, the CSTR (without cell recycle) employed a 45.5-50 min gas retention time, a 0.024-0.031 hour⁻¹ liquid dilution rate, a temperature of 36.5-39.0°C and an agitation rate of 750-1000 rpm. The gas consisted of 65 percent CO, 20 percent H₂, 11 percent CO₂ and 4 percent CH₄. The operating conditions were variable because of fine tuning to yield the maximum ethanol concentration. In this process, the CO gas conversion ranged from 20-60 percent, with higher conversions occurring during the maximum ethanol production. The H₂ conversion remained at about 10 percent. The maximum cell concentration (as optical density) was 4.2, reached at a time of 1040 hours. The optical density (product concentration profile) fell to a level of about 3.5, which corresponded to the time of maximum ethanol production and minimum acetic acid production. The maximum ethanol concentration was about 5 g/L, with a corresponding acetic acid concentration of 0.5 g/L.

Additional experiments performed in the CSTR with isolate O-52 employed a gas retention time of approximately 26 minutes and a liquid retention time of 24 hours. Following a period of product stabilization of 240 hours, the culture shifted from predominantly acetate production to ethanol production. The CO conversions were stable at 85-90 percent and the H₂ conversions were erratic with an average of 20-30 percent. Low H₂ conversions are typical when obtaining relatively high ethanol concentrations in a single CSTR. The ethanol concentration maximized at 20 g/L soon after the switch from acetate to ethanol production, and then stabilized at 10.5-11.0 g/L over the next 250 hours. The corresponding acetate concentration was 2-3 g/L. The dry cell weight concentration was stable throughout the experiments at 1.5-2.0 g/L.

Similarly, *C. ljungdahlii* C-01 is capable of producing ethanol instead of acetic acid as a product from a substrate-containing gas conversion. CSTR experiments were also performed with isolate C-01, which had produced high ratios of ethanol to acetic acid in serum bottle studies. In one such experiment, the gas contained 32 percent H₂, 34 percent CO, 5 percent CH₄ and 29 percent CO₂. The liquid retention time (LRT) was 32 hours and the gas retention time was 14.2 minutes. The reactor had been operating at these conditions for nearly 500 hours. The CO conversion was maintained at about 85 percent, and the H₂ conversion was fairly steady at about 50 percent. The product from the reactor contained 20-24 g/L ethanol and 3-4 g/L acetic acid. The dry cell weight concentration was 1.8-2.4 g/L.

In certain CSTR experiments, the maximum achievable ethanol concentration in a single CSTR was about 25 g/L for both strains, and typical concentrations in the CSTR were about 23 g/L ethanol and about 3.5 g/L acetic acid. Strain C-01 is a bit more tolerant of CO as a substrate, allowing slightly higher dissolved CO concentrations in the liquid phase. Typical specific gas uptake rates for isolate O-52 were 21 mmol CO/gcell per hr and 15 mmol H₂/gcell per hr. The typical specific uptake rates for isolate C-01 were 25 mmol CO/gcell per hr and 10 mmol H₂/gcell per hr. Typical yields of ethanol from the H₂, CO, CO₂ substrates were 90 percent of theoretical. The specific productivities were 0.21 g ethanol/gcell per hr for isolate O-52, and 0.23 g ethanol/gcell per hr for isolate C-01. The isolates are thus nearly interchangeable in their ability to use CO, CO₂ and H₂ to produce ethanol.

Both strains are capable of growing and producing ethanol in the presence of typical (1-2 percent) levels of H₂S and COS. Sulfur is incorporated into cell mass, accounting for about 1 percent of the weight of the cell. NaₛS, an aqueous phase form of H₂S, is also used to help ensure anaerobic conditions inside the reactor.

In summary, the new *C. ljungdahlii* strains O-52 and C-01 produce ethanol by fermentation of CO, CO₂ and H₂, with CO as the preferred substrate. Designation of CO as the preferred substrate means that higher cell yields are obtained on CO, and that higher CO conversions are obtained on gas mixtures containing both CO and H₂. The preferred operating pH ranges for both strains is between pH4.5 and 5.5. The products of the fermentation of CO, CO₂ and H₂ are ethanol and acetic acid. Both strains give very similar product concentrations with nearly identical fermentation conditions of medium composition and concentration, pH, gas and liquid flow rates, agitation rates and gas composition.

Thus the microorganisms of the present invention may be used in a process to produce ethanol by a gaseous substrate fermentation, not only reducing consumption of valuable chemical feedstocks, but also removing hazardous atmospheric pollutants from the waste gas streams of many industries. Previous processes to derive these chemicals biologically were based on fermentation of sugars.

### C. Examples

The following specific examples are submitted to illustrate but not to limit the present invention. Unless otherwise indicated, all parts and percentages in the specification and claims are based upon volume.

### EXAMPLE 1 - PRODUCTION OF ETHANOL FROM OIL REFINERY WASTE GAS

This example is demonstrated on a bench scale continuous conversion system, specifically as described in Example 1 of published PCT Patent Application No. WO98/00558, published January 8, 1998. A mixture of oil refinery waste gases containing about 45 percent CO, 50 percent H₂ and 5 percent CH, is sparged into a 1.0L continuous stirred tank reactor without cell recycle containing anaerobic bacteria isolate O-52 ATCC deposit 55989. Thee refinery waste gases are produced, for example, by mixing streams from the catalyst regenerator and the catalytic cracker, two unit operations commonly found in the refinery. The operating pressure inside the reactor is a few inches of water, and the temperature is 37°C. The gas retention time is 12 minutes and the liquid dilution rate is 0.042 hr¹. The liquid medium contains basal salts and vitamins with a nutrient limitation (no yeast or trypticase) to enhance ethanol production. The operating pH in the reactor is 5.05, and the agitation rate is 1000 rpm. Under these conditions, the CO conversion is 85 percent and the H₂ conversion is 50 percent. The ethanol concentration in the product stream from the reactor is 21 g/L. The acetic acid by-product concentration is 3 g/l.

The product stream from the fermenter is sent to distillation for product recovery. The bottoms from the distillation column containing acetic acid and water is sent back to the fermenter as water recycle. The overhead product from distillation containing 95 percent ethanol is sent to an adsorption unit to produce neat ethanol.

9

### EXAMPLE 2 - PRODUCTION OF ETHANOL FROM WASTE GAS USING BRI ISOLATE C-01

This example is demonstrated on a bench scale continuous conversion system, specifically as described in Example 1 of published PCT Patent Application No. WO98/00558, published January 8, 1998. Carbon black waste gas containing about 14 percent CO, 17 percent H₂, and 4 percent CO₂ in N₂ is sparged into a 1.01L continuous stirred tank reactor without cell recycle operating at 1.58 atm, 37°C and containing *Clostridium ljungdahlii* isolate C-01 [ATCC Accession No. 55988], which is capable of producing ethanol as the final product. A trickle bed reactor is a column packed with a commercial packing such as Raschig rings or Berl saddles in which liquid and gas are contacted with each other due to flow through the column. In the present example, the liquid and gas both enter the column from the top in a concurrent fashion, although countercurrent flow (gas entering the bottom, liquid entering the top) is possible. The gas retention time is maintained at 0.46 minute and the liquid medium dilution rate is 0.57 hour⁻¹. The nutrient mixture fed to the culture was as follows:
1. 80.0 ml of a salt, composed of:

| | |
|---|---|
| KH₂PO₄ | 3.00 g/L |
| K₂HPO₄ | 3.00 g/L |
| (NH₄)₂SO₄, | 6.00 g/L |
| NaCl | 6.00 g/L |
| MgSO₄.2H₂O | 1.25 g/L |

2. 1.0 g of yeast extract
3. 1.0 g of trypticase
4. 3.0 ml of PFN trace metal solution (Pfenning] containing:

| | |
|---|---|
| FeCl₂ * 4H₂O | 1500 mg |
| ZnSO₄ * 7H₂O | 100 mg |
| MnCl₂ * 4H₂O | 30 mg |
| H₃BO₃ | 300 mg |
| CoCl₂ * 6H₂O | 200 mg |
| CuCl₂ * H₂O | 10 mg |
| NiCl₂ * 6H₂O | 20 mg |
| NaMoO₄ * 2H₂O | 30 mg |
| Na₂ SeO₃ | 10 mg |
| Distilled water | 1000 ml |

5. 10.0 ml of B vitamins:

| | |
|---|---|
| Pyridoxal HCl | 10 mg |
| Riboflavin | 50 mg |
| Thiamine HCl | 50 mg |
| Nicotinic acid | 50 mg |
| Ca-D-Pantotheinate | 50 mg |
| Lipoic Acid | 60 mg |
| P-aminobenzoic acid | 50 mg |
| Folic acid | 20 mg |
| Biotin | 20 mg |
| Cyanocobalamin | 50 mg |
| Distilled water | 1000 ml |

6. 0.5 g of Cysteine HCl
7. 0.6 g of CaCl₂.2H₂O
8. 2.0 g of NaHCO₃
9. 1.0 ml of Resazurin (0.01%)
10. 920.0 ml of distilled water

Agitation in the reactor is provided by liquid recirculation, using a recirculation rate of 60 gpm. The operating pH in the reactor is 5.05. Under these conditions, the CO conversion is 57 percent and the H₂ conversion is 58 percent. A hollow fiber unit is used to maintain a cell concentration of 13.6 g/L inside the reactor.

The product from the reactor is 20 g/L ethanol and 3 g/L acetic acid.

Thus, it will be appreciated that as a result of the microorganisms of the present invention, a highly effective improved process for converting waste gases to ethanol is accomplished. It is contemplated and will be apparent to those skilled in the art from the preceding description that modifications and/or changes may be made in the illustrated embodiments without departure from the present invention. Accordingly, it is expressly intended that the foregoing description and accompanying drawings are illustrative of preferred embodiments only, and are not limiting, and that the scope of the present invention be determined by reference to the appended claims.

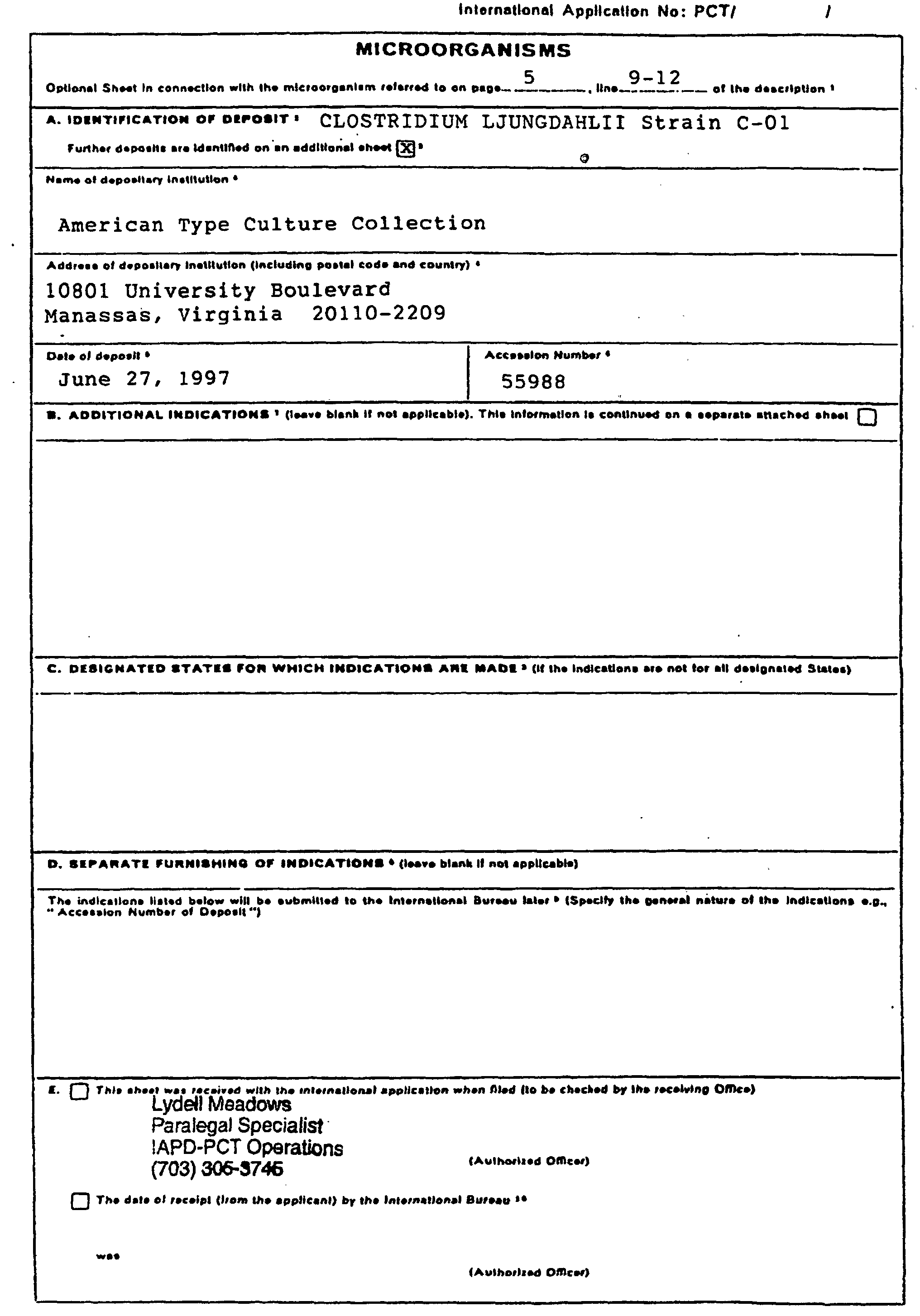

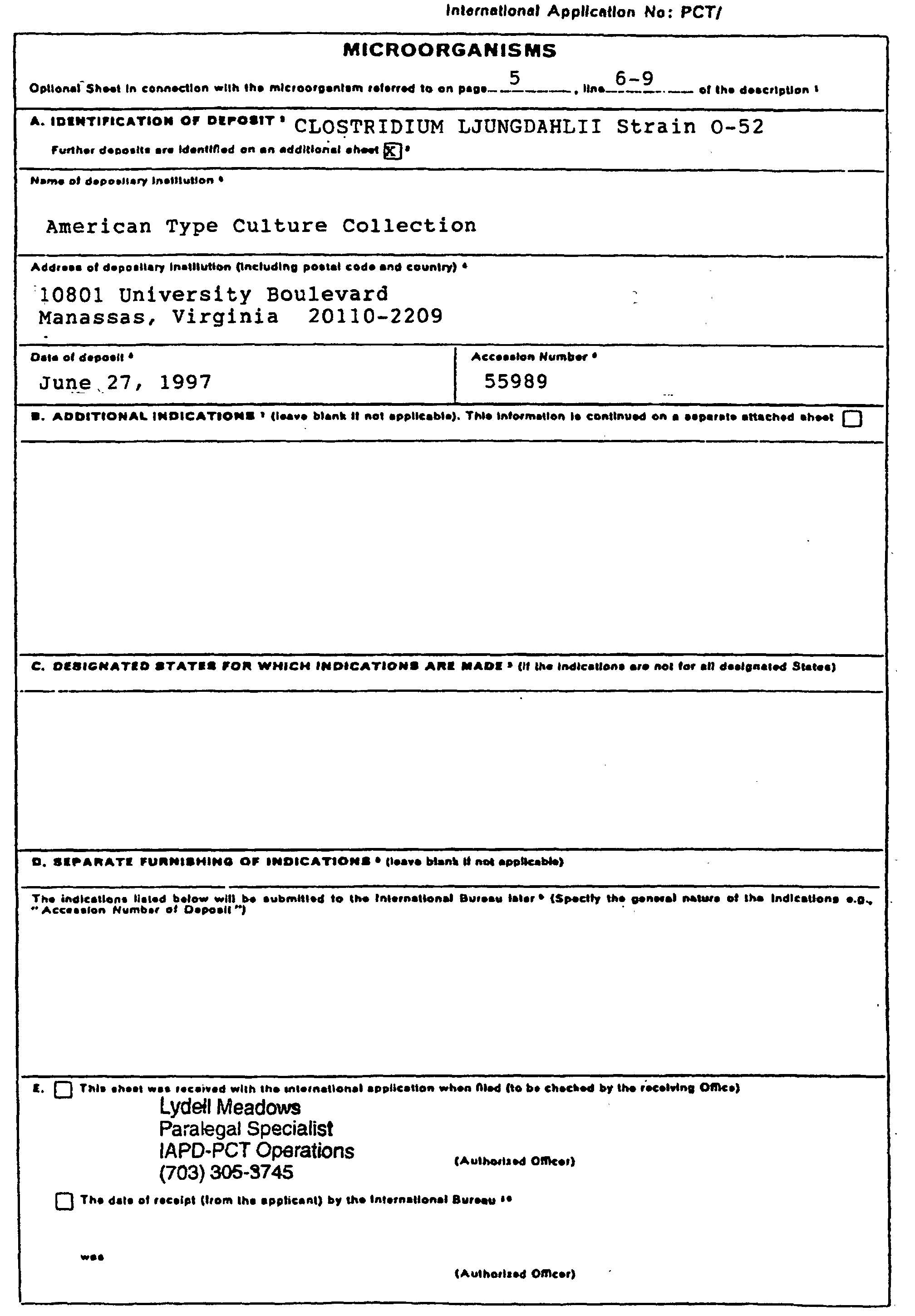

## Claims

1. An isolated *C. ljungdahlii* strain having the characteristics of strain C-01, ATCC Accession No. 55988, and **characterized by** the ability to produce ethanol by anaerobic fermentation conversion of a substrate-containing gas.

2. An isolated *C. ljungdahlii* strain having the characteristics of strain O-52, ATCC Accession No. 55989, and **characterized by** the ability to produce ethanol by anaerobic fermentation conversion of a substrate-containing gas.

3. A process for producing ethanol, comprising the steps of:
(a) fermenting a substrate-containing gas anaerobically with *C. ljungdahlii* O-52, ATCC Accession No. 55989, or *C. ljungdahlii* C-01, ATCC Accession No. 55988 in an aqueous nutrient media in a bioreactor to produce a broth;
(b) removing continuously a portion of said broth from said bioreactor, and
(c) recovering ethanol therefrom.

4. The process according to claim 3 wherein the substrate-containing gas contains carbon monoxide and water.

5. The process according to claim 3 wherein the substrate-containing gas contains carbon dioxide and hydrogen.

## Patentansprüche

1. Isolierter *C. ljungdahlii*-Stamm mit den Eigenschaften des Stamms C-01, ATCC-Zugangsnr.55988, **dadurch gekennzeichnet, daß** er die Fähigkeit besitzt, durch Umsetzung eines substrathaltigen Gases mittels anaerober Fermentation Ethanol zu produzieren.

2. Isolierter *C. ljungdahlii*-Stamm mit den Eigenschaften des Stamms O-52, ATCC-Zugangsnr.55989, **dadurch gekennzeichnet, daß** er die Fähigkeit besitzt, durch Umsetzung eines substrathaltigen Gases mittels anaerober Fermentation Ethanol zu produzieren.

3. Verfahren zur Herstellung von Ethanol, bei dem man:
(a) ein substrathaltiges Gas anaerobisch mit *C. ljungdahlii* O-52, ATCC-Zugangsnr. 55989, oder *C. ljungdahlii* C-01, ATCC-Zugangsnr. 55988, in einem wäßrigen Nährmedium in einem Bioreaktor zur Herstellung einer Brühe fermentiert;
(b) kontinuierlich einen Teil der Brühe aus dem Bioreaktor entnimmt und
(c) daraus Ethanol gewinnt.

4. Verfahren nach Anspruch 3, wobei das substrathaltige Gas Kohlenmonoxid und Wasser enthält.

5. Verfahren nach Anspruch 3, wobei das substrathaltige Gas Kohlendioxid und Wasserstoff enthält.

## Revendications

1. Souche de *C. ljungdahlii* isolée ayant les caractéristiques de la souche C-01, numéro d'accession ATCC 55988, et **caractérisée par** la capacité de produire de l'éthanol par conversion en fermentation anaérobique d'un gaz contenant un substrat.

2. Souche de *C. ljungdahlii* isolée ayant les caractéristiques de la souche O-52, numéro d'accession ATCC 55989, et **caractérisée par** la capacité de produire de l'éthanol par conversion en fermentation anaérobique d'un gaz contenant un substrat.

3. Procédé pour produire de l'éthanol, comprenant les étapes de:
(a) fermentation anaérobie d'un gaz contenant un substrat au moyen de *C. ljungdahlii* O-52, numéro d'accession ATCC 55989, ou de *C. ljungdahlii* C-01, numéro d'accession ATCC 55988, dans un milieu nutritif aqueux dans un bioréacteur afin de produire un bouillon;
(b) enlèvement continu d'une portion dudit bouillon dudit bioréacteur; et
(c) récupération de l'éthanol à partir de celle-ci.

4. Procédé selon la revendication 3, dans lequel le gaz contenant un substrat contient du monoxyde de carbone et de l'eau.

5. Procédé selon la revendication 3, dans lequel le gaz contenant un substrat contient du dioxyde de carbone et de l'hydrogène.
